# EUROPÄISCHE PATENTSCHRIFT

(11) **EP 1 982 731 B1**
(45) Veröffentlichungstag und Bekanntmachung des Hinweises auf die Patenterteilung: **16.06.2010**
(21) Anmeldenummer: 07007840.7
(22) Anmeldetag: 17.04.2007
(51) Int. Cl.: A61K 47/00, A61K 36/185

(54) **Verfahren zur Herstellung von lagerstabilen Lösungen aus Pelargonium-Extrakten**
Method for producing storage stable solutions of pelargonium extracts
Procédé de fabrication de solutions des extraits de pélargonium stables au stockage

(43) Veröffentlichungstag der Anmeldung: 22.10.2008
(73) Patentinhaber: Dr. Willmar Schwabe GmbH & Co. KG, 76227 Karlsruhe (DE)
(72) Erfinder: Germer, Stefan, 76227 Karlsruhe (DE); Hauer, Hermann, 76228 Karlsruhe (DE); Thöle, Marc, 76149 Karlsruhe (DE)
(74) Vertreter: Adam, Holger

(56) Entgegenhaltungen:
- WO-A-2004/000045
- WO-A-2006/002837
- WO-A1-98/58656
- O.A. NEUMÜLLER: "Römpps Chemie-Lexikon, Band 5" 1987, FRANCKH'SCHE VERLAGSHANDLUNG , STUTTGART * Seite 3550 *

## Beschreibung

Die Erfindung betrifft Verfahren zur Herstellung von lagerstabilen Lösungen aus Pelargonium-Extrakten, **dadurch gekennzeichnet, dass** die Sauerstoffmenge bei atmosphärischem Druck im Kopfraum des zur Lagerung der Lösung aus Pelargonium-Extrakten verwendeten Gebindes auf maximal 0,025 Volumenteile (bevorzugt 0,015 Volumenteile, besonders bevorzugt 0,005 Volumenteile) pro Volumenteil der Lösung reduziert wird, wobei der Gehalt an Proanthocyanidinen und an 2H-1-Benzopyran-2-onen nach 9 Monaten Lagerung bei 25 °C und einer relativen Luftfeuchtigkeit von 60% um jeweils maximal 10 Gew.-% (bevorzugt maximal 7 Gew.-%, besonders bevorzugt maximal 5 Gew.-%, insbesondere maximal 3 Gew.-%) verringert ist, wobei die Sauerstoffmenge a) durch Begasen mit einem Schutzgas und/oder b) durch ein verringertes Volumen des Kopfraums und/oder c) durch Zusatz eines Sauerstoff-entfernenden Mittels reduziert wird, wobei das Sauerstoff entfernende Mittel eine oder mehrere Substanzen enthält, ausgewählt aus der Gruppe bestehend aus Ascorbinsäure, Salzen der Ascorbinsäure, Ascorbinsäure-Derivaten, Metallen oder Metallsalzen in niedrigen Oxidationsstufen und oxidierbaren Polymeren, wobei die Salze der Ascorbinsäure ausgewählt sind aus der Gruppe umfassend Natriumascorbat, Kaliumascorbat und Calciumascorbat, wobei die Ascorbinsäure-Derivate ausgewählt sind aus der Gruppe umfassend Ester der Ascorbinsäure mit Fettsäuren, wobei die Metalle oder Metallsalze in niedrigen Oxidationsstufen ausgewählt sind aus der Gruppe umfassend Eisen, Eisen(II)-oxid, Eisen(II)-hydroxid und Eisen(II)-chlorid, und wobei das oxidierbare Polymer ein kondensiertes Polymer aus m-Xylylendiamin und Adipinsäure ist.

Bei den in dem erfindungsgemäßen Verfahren verwendeten Lösungen aus Pelargonium-Extrakten kann es sich zum einen um unmittelbar bei der Extraktherstellung erhaltene Flüssigextrakte oder um Lösungen von Trockenextrakten oder Spissumextrakten in pharmazeutisch verträglichen Lösungsmitteln, insbesondere Wasser und wässrigen Alkoholen und Polyolen, wie Glycerol und Ethanol, sowie deren Gemische handeln.

Die ebenfalls als Lösungen aus Pelargonium-Extrakten verwendbaren Pelargonium-Flüssigextrakte können nach an sich bekannten Verfahren hergestellt werden. Grundsätzlich können im erfindungsgemäßen Verfahren jegliche Pelargonium-Flüssigextrakte verwendet werden, die Proanthocyanidine und 2H-1-Benzopyran-2-one enthalten. Die in dem erfindungsgemäßen Verfahren verwendeten Lösungen aus Perlargonium-Extrakten in Form von Flüssigextrakten können beispielsweise gewonnen werden, indem zunächst getrocknete und zerkleinerte Wurzeln von Pelargonium sidoides und / oder Pelargonium reniforme mit einem Lösungsmittel ausgewählt aus der Gruppe umfassend Wasser, wässrige Alkohole, wässrige Polyole und Gemische davon, in herkömmlicher Weise, beispielsweise bei Temperaturen von 10 bis 100°C, extrahiert werden. Der Drogenrückstand wird gegebenenfalls leicht ausgepresst und der rohe Extrakt wird gegebenenfalls filtriert.

Bevorzugt erfolgt die Herstellung des Pelargonium-Flüssigextrakts durch Perkolation mit einem wässrig-ethanolischen Lösungsmittel, gegebenenfalls nach einem zuvor erfolgten Anmaischen mit einem wässrig-ethanolischen Lösungsmittel entsprechend der EP 1 429 795.

Weitere geeignete Pelargonium-Flüssigextrakte sind beispielsweise auch in der DE 10 2004 063910, insbesondere im Absatz [0017] und den Beispiele 3 und 4 beschrieben. Der Offenbarungsgehalt der beiden letztgenannten Druckschriften ist bezüglich der Herstellung der Pelargonium-Flüssigextrakte ausdrücklich unter Bezugnahme hierin umfasst. Die Extraktlösung kann dabei entweder direkt bei der Herstellung anfallen oder durch Auflösen eines geeigneten Trockenextrakts gewonnen werden.

Die WO 98/58656 offenbart lagerstabile Zusammensetzungen mit Citrusgeschmack, umfassend Citral oder Citralderivate als Geschmacksstoff und einen Pflanzenextrakt als Stabilisierungsmittel. Der Pflanzenextrakt hemmt die Bildung von p-Methylacetophenon, wobei die Entwicklung von Geschmacks- und Geruchsabweichungen verhindert wird und die Lagerstabilität der Zusammensetzung verbessert wird.

Die WO 2004/000045 beschreibt flüssige Zusammensetzungen, die nicht verdaubare Oligosacharide und Katechine aus grünem Tee umfassen.

Extrakte aus Pelargonium-Arten, insbesondere aus Pelargonium sidoides und/oder Pelargonium reniforme können in Arzneimitteln oder Lebensmitteln verwendet werden und werden in der Regel als feste oder flüssige Darreichungsformen peroral eingenommen. Wesentliche Bestandteile dieser Extrakte sind dabei Proanthocyanidine und substituierte 2H-1-Benzopyran-2-one (Cumarine).

Lösungen aus Pelargonium-Extrakten, wie flüssige galenische Darreichungsformen, die diese Extrakte aus Pelargonium sidoides und / oder reniforme in Lösung enthalten, weisen jedoch den Nachteil einer unzureichenden Lagerstabilität auf. Bei Lagerung wird eine Abnahme des Gehalts an Proanthocyanidinen und an 2H-1-Benzopyran-2-onen festgestellt.

Aufgabe der vorliegenden Erfindung ist daher, lagerstabile Lösungen aus Pelargonium-Extrakten zur Verfügung zu stellen. Dabei dürfen der Gehalt an Proanthocyanidinen und der Gehalt an 2H-1-Benzopyran-2-onen während der gewünschten Lagerzeit bei definierten Bedingungen um jeweils maximal 10 % abnehmen. Insbesondere sollen die Gehalte bei Lagerung bei einer Temperatur von 25 °C und einer relativen Luftfeuchtigkeit von 60 % innerhalb von neun Monaten um jeweils maximal 10 %, bevorzugt maximal 7 %, besonders bevorzugt maximal 5 % und insbesondere maximal 3 %, jeweils bezogen auf das Gewicht, abnehmen.

Diese Aufgabe wird erfindungemäß gelöst durch ein Verfahren zur Herstellung von Lösungen aus Pelargonium-Extrakten, **dadurch gekennzeichnet, dass** die Sauerstoffmenge bei atmosphärischem Druck im Kopfraum des zur Lagerung der Lösung aus Pelargonium-Extrakten verwendeten Gebindes auf maximal 0,025 Volumenteile pro Volumenteil der Lösung reduziert wird, wobei der Gehalt an Proanthocyanidinen und an 2H-1-Benzopyran-2-onen nach 9 Monaten Lagerung bei 25°C und einer relativen Luftfeuchtigkeit von 60 % um jeweils maximal 10 Gew.-% verringert ist, wobei die Sauerstoffmenge a) durch Begasen mit einem Schutzgas und/oder b) durch ein verringertes Volumen des Kopfraums und/oder c) durch Zusatz eines Sauerstoff-entfernenden Mittels reduziert wird, wobei das Sauerstoff entfernende Mittel eine oder mehrere Substanzen enthält, ausgewählt aus der Gruppe bestehend aus Ascorbinsäure, Salzen der Ascorbinsäure, Ascorbinsäure-Derivaten, Metallen oder Metallsalzen in niedrigen Oxidationsstufen und oxidierbaren Polymeren, wobei die Salze der Ascorbinsäure ausgewählt sind aus der Gruppe umfassend Natriumascorbat, Kaliumascorbat und Calciumascorbat, wobei die Ascorbinsäure-Derivate ausgewählt sind aus der Gruppe umfassend Ester der Ascorbinsäure mit Fettsäuren, wobei die Metalle oder Metallsalze in niedrigen Oxidationsstufen ausgewählt sind aus der Gruppe umfassend Eisen, Eisen(II)-oxid, Eisen(II)-hydroxid und Eisen(II)-chlorid, und wobei das oxidierbare Polymer ein kondensiertes Polymer aus m-Xylylendiamin und Adipinsäure ist.

Unter Proanthocyanidinen sind dabei mono-, oligo- und polymere Flavonderivate zu verstehen, die aus Flavan-3-ol-Einheiten aufgebaut sind, bevorzugt aus Gallocatechin, Epigallocatechin, Catechin und Epicatechin und üblicherweise mit Gruppenbestimmungsmethoden, z. B. photometrisch nach Folin-Ciocalteu erfasst werden.

In Pelargonium-Arten enthaltene und zu stabilisierende 2H-1-Benzopyran-2-one sind in 5- bis 8-Position zwei-bis vierfach mit Hydroxy, Methoxy und/oder Sulfooxy substituierte 2H-1-Benzopyran-2-one. Die Quantifizierung erfolgt dabei typischerweise mittels HPLC mit UV-Detektion, wobei eine oder mehrere Leitsubstanzen wie z. B. 6,8-Bis(sulfooxy)-7-hydroxy-2H-1-benzopyran-2-on (Verbindung I) oder 7-Hydroxy-5,6-dimethoxy-8-sulfooxy-2H-1-benzopyran-2-on (Verbindung II) zur Bestimmung ausgewählt werden.

Überraschend wurde festgestellt, dass derartige lagerstabile Lösungen aus Pelargonium-Extrakten erhalten werden, wenn das Sauerstoffvolumen im Kopfraum der zur Lagerung verwendeten Gebinde begrenzt bzw. reduziert wird. Besonders überraschend ist, dass diese Stabilisierung auch im Fall der 2H-1-Benzopyran-2-one gelingt, da diese nicht als sauerstoffempfindlich gelten.

Die 2H-1-Benzopyran-2-on-Grundstruktur ist in neutralem oder schwach saurem Medium üblicherweise sehr stabil, so dass bei den vorgenannten substituierten 2H-1-Benzopyran-2-onen eine Instabilität allenfalls durch hydrolytische Abspaltung der Sulfatreste erwartet werden konnte. Somit sollte beispielsweise durch Einstellung des pH-Wertes die Hydrolyse zu unterbinden sein, nicht jedoch durch die erfindungsgemäßen Maßnahmen eine Stabilisierung der Verbindungen I und II zu erreichen sein. Es ist deshalb besonders überraschend, dass mit dem erfindungsgemäßen Verfahren die Stabilisierung auch im Fall der 2H-1-Benzopyran-2-one gelingt.

Das erfindungsgemäße Verfahren zur Reduktion des Sauerstoffgehalts umfasst die Auswahl eines Gebindes mit entsprechend kleinem Kopfraum und / oder das Verdrängen des Sauerstoffs mittels Schutzbegasung und / oder das Entfernen des Sauerstoffs aus dem Kopfraum des Gebindes durch ein Sauerstoffentfernendes Mittel ("oxygen scavenger"), wobei dieses Mittel aus einer oder mehreren Substanzen bestehen kann und so beschaffen ist, dass es nicht oder nur in unwesentlichem Ausmaß mit Inhaltsstoffen der Lösung aus Pelargonium-Extrakten reagiert.

Erfindungsgemäß sind das Kopfraumvolumen der Gebinde und / oder die Bedingungen der Schutzbegasung so zu wählen, dass das Sauerstoffvolumen im Kopfraum bei atmosphärischem Druck maximal 0,025 Volumenteile (bevorzugt 0,015 Volumenteile, besonders bevorzugt 0,005 Volumenteile) Sauerstoff pro Volumenteil der Lösung aus Pelargonium-Arten beträgt. Als Schutzgas kommen insbesondere Stickstoff, Kohlendioxid oder Edelgase wie z. B. Argon, auch in Mischungen, in Betracht.

Sauerstoff-entfernende Mittel ("oxygen scavenger") enthalten eine oder mehrere Substanzen, die allein und/oder in Kombination in der Lage sind, im Kopfraum des Gebindes eingeschlossenen Sauerstoff durch Adsorption und / oder Absorption und / oder chemische Umsetzung zu entfernen. Die Zusammensetzung des Sauerstoff-entfernenden Mittels ist erfindungsgemäß so zu wählen, dass der Sauerstoffgehalt im Gebinde-Kopfraum auf maximal 0,025 Volumenteile (bevorzugt 0,015 Volumenteile, besonders bevorzugt 0,005 Volumenteile) Sauerstoff pro Volumenteil der Lösung aus Pelargonium-Extrakten reduziert wird.

Als Bestandteil der Sauerstoff entfernenden Mittel sind beispielsweise folgende Substanzen geeignet: Ascorbinsäure, Salze der Ascorbinsäure wie Natriumascorbat, Kaliumascorbat oder Calciumascorbat, Ester der Ascorbinsäure mit Fettsäuren wie Palmitin- oder Stearinsäure, Metalle oder Metallsalze in niedrigen Oxidationsstufen wie Eisen, Eisen(II)-oxid, Eisen(II)-hydroxid oder Eisen(II)-chlorid oder oxidierbare Polymere wie MXD6, ein kondensiertes Polymer aus m-Xylylendiamin und Adipinsäure.

Aus der Literatur sind zahlreiche mögliche Ausführungsformen für geeignete Sauerstoff-entfernende Mittel bekannt.

Die JP3014481 offenbart beispielsweise einen sauerstoffpermeablen, Eisen und gegebenenfalls weitere Substanzen enthaltenden Beutel mit geringer Feuchtigkeitsdurchlässigkeit, der zum Entfernen von Sauerstoff aus mit Flüssigkeiten befüllten Gebinden eingesetzt werden kann.

Aus der JP2003081353 sind adhäsive Folien mit mehrschichtigem Aufbau, darunter eine Sauerstoff aufnehmende Schicht, bekannt, die an der Innenseite des zur Aufbewahrung eines sauerstoffempfindlichen Gutes verwendeten Packmittels befestigt werden können.

Schraubverschlüsse mit Sauerstoff aufnehmenden Einlagen sind beispielsweise in der EP1742850 beschrieben.

Eine weitere mögliche Ausführungsform stellen mehrschichtige Kunststoffbehältnisse dar, bei denen der Sauerstoff durch eine Schicht aus mindestens einem oxidierbaren Polymer gebunden wird, wie beispielsweise in der W02005 014410 offenbart.

Die Bestimmung der Proanthocyanidine (= Gesamtphenole) und der 2H-1-Benzopyran-2-one I und II ist im Folgenden angegeben:
Bestimmung der Proanthocyanidine nach Folin-Ciocalteu:
   Die Bestimmung der Proanthocyanidine erfolgt in Analogie zur Arzneibuchmethode für Gerbstoffe (DAB 2000) photometrisch nach Umsetzung mit Molybdat-Wolframat-Reagenz. Dazu wird der Extrakt in wässrigem Ethanol gelöst, mit Natriumcarbonat-Lösung alkalisiert und mit Molybdat-Wolframat-Reagenz versetzt. Nach Zentrifugation wird die Extinktion der überstehenden Lösung bei 720 nm gegen Wasser gemessen. Die Berechnung erfolgt auf Epicatechin.
Bestimmung der 2H-1-Benzopyran-2-one I und II:
   Die Bestimmung der Verbindungen I und II erfolgt mittels HPLC über eine RP-18 Säule. Als mobile Phase wird ein Acetonitril / Wasser / Phosphorsäure-Gradient (10:990:4 → 205:795:4) verwendet. Detektiert wird im UV bei 330 nm. Die Berechung der einzelnen Cumarin-Peaks erfolgt als Scopoletin.

Zur Sauerstoff-Bestimmung im Gebinde-Kopfraum kommt eine Standardmethode zum Einsatz (GC über eine Molekularsiebsäule mit Wärmeleitfähigkeitsdetektion).

Prozentangaben beziehen sich in der vorstehenden Beschreibung und in den nachfolgenden Beispielen, soweit nichts anderes angegeben ist, auf das Gewicht.

### Beispiele

In den Beispielen 1 bis 4 wurde folgender ethanolisch-wässrige Auszug eingesetzt:
Gemahlene Wurzel von Pelargonium sidoides wurden mit der doppelten Gewichtsmenge Ethanol (35 Gew. %) versetzt und 20 h bei Raumtemperatur gelagert. Danach wurde die Mischung für 10 h mit der achtfachen Gewichtsmenge Ethanol (6 Gew. %) perkoliert und anschließend filtriert.

### Beispiel 1: (Vergleichsbeispiel)

Eine Lösung, enthaltend 80 Gew. % des obigen ethanolisch-wässrigen Auszugs aus Pelargonium sidoides und 20 Gew. % Glycerol, wird unter normaler Atmosphäre (21 % Sauerstoff) in Braunglasflaschen abgefüllt. Das Füllvolumen beträgt 20 ml, das Kopfraumvolumen der Flaschen beträgt 5 ml. Die Flaschen werden mit einem Tropfeinsatz versehen und mit einem Schraubverschluss dicht verschlossen. Der Sauerstoffgehalt im Kopfraum beträgt 0,053 Volumenteile Sauerstoff pro Volumenteil der Lösung. Nach 9 Monaten Lagerung bei 25 °C / 60 % relative Luftfeuchtigkeit ist der mit der Methode nach Folin-Ciocalteu bestimmte Gehalt der Lösung an Proanthocyanidinen sowie der mittels HPLC bestimmte Gehalt an Verbindungen I und II reduziert.

| Substanz | Gehalt bezogen auf den Ausgangswert |
|---|---|
| Proanthocyanidine | 85,4 % |
| Verbindung I | 89,0 % |
| Verbindung II | 90,9% |

### Beispiel 2: (Verringerung des Kopfraumvolumens)

Eine Lösung, enthaltend 80 Gew. % des obigen ethanolisch-wässrigen Auszugs aus Pelargonium sidoides und 20 Gew. % Glycerol, wird unter normaler Atmosphäre (21 % Sauerstoff) in Braunglasflaschen abgefüllt. Das Füllvolumen beträgt 23 ml, das Kopfraumvolumen der Flaschen beträgt 2 ml. Die Flaschen werden unter normaler Atmosphäre mit einem Tropfeinsatz versehen und mit einem Schraubverschluss dicht verschlossen. Der Sauerstoffgehalt im Kopfraum beträgt 0,018 Volumenteile Sauerstoff pro Volumenteil der Lösung. Nach 9 Monaten Lagerung bei 25 °C / 60 % relative Luftfeuchtigkeit ist der mit der Methode nach Folin-Ciocalteu bestimmte Gehalt der Lösung an Proanthocyanidinen sowie der mittels HPLC bestimmte Gehalt an Verbindungen I und II deutlich weniger reduziert als in Beispiel 1 (Vergleichsbeispiel).

| Substanz | Gehalt bezogen auf den Ausgangswert |
|---|---|
| Proanthocyanidine | 94,0 % |
| Verbindung I | 91,3 % |
| Verbindung II | 94,5 % |

### Beispiel 3: (Schutzbegasung mit Stickstoff)

Eine Lösung, enthaltend 80 Gew. % des obigen ethanolisch-wässrigen Auszugs aus Pelargonium sidoides und 20 Gew. % Glycerol, wird unter normaler Atmosphäre in Braunglasflaschen abgefüllt. Das Füllvolumen beträgt 20 ml, das Kopfraumvolumen der Flaschen beträgt 5 ml. Die Flaschen werden mit Stickstoff überschichtet, mit einem Tropfeinsatz versehen und mit einem Schraubverschluss dicht verschlossen. Der nach dem Verschließen gemessene Sauerstoffgehalt im Kopfraum der Flaschen beträgt 7 %. Der Sauerstoffgehalt im Kopfraum beträgt 0,018 Volumenteile Sauerstoff pro Volumenteil der Lösung. Nach 9 Monaten Lagerung bei 25 °C / 60 % relative Luftfeuchtigkeit ist der mit der Methode nach Folin-Ciocalteu bestimmte Gehalt der Lösung an Proanthocyanidinen sowie der mittels HPLC bestimmte Gehalt an Verbindungen I und II deutlich weniger reduziert als in Beispiel 1 (Vergleichsbeispiel).

| Substanz | Gehalt bezogen auf den Ausgangswert |
|---|---|
| Proanthocyanidine | 99,0 % |
| Verbindung I | 93,5 % |
| Verbindung II | 93,3 % |

### Beispiel 4: (Abfüllung unter Stickstoff-Begasung in Ampullen)

Eine Lösung, enthaltend 80 Gew. % des obigen ethanolisch-wässrigen Auszugs aus Pelargonium sidoides und 20 Gew. % Glycerol, wird unter Stickstoff-Begasung in Ampullen abgefüllt. Das Füllvolumen beträgt 5 ml. Die Ampullen werden zugeschmolzen. Das Kopfraumvolumen der Ampullen beträgt 1 ml. Der nach dem Verschließen gemessene Sauerstoffgehalt im Kopfraum der Ampullen beträgt 1 %. Der Sauerstoffgehalt im Kopfraum beträgt 0,002 Volumenteile Sauerstoff pro Volumenteil der Lösung. Nach 9 Monaten Lagerung bei 25 °C / 60 % relative Luftfeuchtigkeit ist der mit der Methode nach Folin-Ciocalteu bestimmte Gehalt der Lösung an Proanthocyanidinen sowie der mittels HPLC bestimmte Gehalt an Verbindungen I und II bezogen auf den Ausgangswert vergleichbar bzw. kaum reduziert.

| Substanz | Gehalt bezogen auf den Ausgangswert |
|---|---|
| Proanthocyanidine | 100,9% |
| Verbindung I | 97,5 % |
| Verbindung II | 98,5 % |

In den erfindungsgemäßen Beispielen 2, 3 und 4 (Sauerstoffgehalt im Kopfraum weniger als 0,025 Volumenteile Sauerstoff pro Volumenteil der Lösung) liegen die Gehalte der Proanthocyanidine sowie der Verbindungen I und II nach 9 Monaten Lagerung bei 25 °C / 60 % relative Luftfeuchte jeweils bei mehr als 90 % und entsprechen damit den gewünschten Anforderungen an die Stabilität. Dagegen liegen zwei der drei entsprechenden Gehalte im Vergleichsbeispiel (Beispiel 1, Sauerstoffgehalt im Kopfraum mehr als 0,025 Volumenteile Sauerstoff pro Volumenteil der Lösung) unter 90 % und entsprechen damit nicht den gewünschten Anforderungen an die Stabilität.

## Patentansprüche

1. Verfahren zur Herstellung von lagerstabilen Lösungen aus Pelargonium-Extrakten, **dadurch gekennzeichnet, dass** die Sauerstoffmenge bei atmosphärischem Druck im Kopfraum des zur Lagerung der Lösung aus Pelargonium-Extrakten verwendeten Gebindes auf maximal 0,025 Volumenteile pro Volumenteil der Lösung reduziert wird, wobei der Gehalt an Proanthocyanidinen und an 2H-1-Benzopyran-2-onen nach 9 Monaten Lagerung bei 25 °C und einer relativen Luftfeuchtigkeit von 60% um jeweils maximal 10 Gew.-% verringert ist,
wobei die Sauerstoffmenge a) durch Begasen mit einem Schutzgas und/oder b) durch ein verringertes Volumen des Kopfraums und/oder c) durch Zusatz eines Sauerstoff-entfernenden Mittels reduziert wird, wobei das Sauerstoff entfernende Mittel eine oder mehrere Substanzen enthält, ausgewählt aus der Gruppe bestehend aus Ascorbinsäure, Salzen der Ascorbinsäure, Ascorbinsäure-Derivaten, Metallen oder Metallsalzen in niedrigen Oxidationsstufen und oxidierbaren Polymeren,
wobei die Salze der Ascorbinsäure ausgewählt sind aus der Gruppe umfassend Natriumascorbat, Kaliumascorbat und Calciumascorbat,
wobei die Ascorbinsäure-Derivate ausgewählt sind aus der Gruppe umfassend Ester der Ascorbinsäure mit Fettsäuren,
wobei die Metalle oder Metallsalze in niedrigen Oxidationsstufen ausgewählt sind aus der Gruppe umfassend Eisen, Eisen(II)-oxid, Eisen(II)-hydroxid und Eisen(II)-chlorid, und
wobei das oxidierbare Polymer ein kondensiertes Polymer aus m-Xylylendiamin und Adipinsäure ist.

2. Verfahren nach Anspruch 1, wobei die 2H-1-Benzopyran-2-one ausgewählt sind aus 6,8-Bis(sulfooxy)-7-hydroxy-2H-1-benzopyran-2-on (Verbindung I) und 7-Hydroxy-5,6-dimethoxy-8-sulfooxy-2H-1-benzopyran-2-on (Verbindung II).

3. Verfahren nach Anspruch 1 oder 2, wobei das Schutzgas ausgewählt ist aus der Gruppe bestehend aus Stickstoff, Edelgasen wie Argon, und Kohlendioxid, sowie Mischungen dieser Gase.

4. Verfahren nach Anspruch 1, wobei die Ascorbinsäure-Derivate ausgewählt sind aus der Gruppe umfassend Ester der Ascorbinsäure mit Palmitin- oder Stearinsäure.

5. Verfahren nach einem der Ansprüche 1 bis 4, wobei der Pelargonium Extrakt aus Pelargonium sidoides und/oder Pelargonium reniforme gewonnen wird.

6. Verfahren nach einem der Ansprüche 1 bis 5, wobei die Lösung aus Pelargonium-Extrakten eine wässrige Lösung oder eine Lösung in Wasser und einem oder mehreren Alkoholen und/oder Polyolen ist.

7. Verfahren nach Anspruch 6, wobei die Lösung aus Pelargonium-Extrakten eine wässrig-ethanolische Lösung, eine Lösung in einem Gemisch aus Wasser und Glycerol oder Wasser, Glycerol und Ethanol ist.

8. Verfahren nach einem der Ansprüche 1 bis 7, wobei die Sauerstoffmenge bei atmosphärischem Druck im Kopfraum des zur Lagerung der Lösung aus Pelargonium-Extrakten verwendeten Gebindes auf maximal 0,015 Volumenteile, pro Volumenteil der Lösung reduziert wird.

9. Verfahren nach einem der Ansprüche 1 bis 7, wobei die Sauerstoffmenge bei atmosphärischem Druck im Kopfraum des zur Lagerung der Lösung aus Pelargonium-Extrakten verwendeten Gebindes auf maximal 0,005 Volumenteile, pro Volumenteil der Lösung reduziert wird.

10. Verfahren nach einem der Ansprüche 1 bis 9, wobei der Gehalt an Proanthocyanidinen und 2H-1-Benzopyran-2-one nach 9 Monaten Lagerung bei 25°C und einer relativen Luftfeuchtigkeit von 60 % um jeweils maximal 7 Gew.-% verringert ist.

11. Verfahren nach einem der Ansprüche 1 bis 9, wobei der Gehalt an Proanthocyanidinen und 2H-1-Benzopyran-2-one nach 9 Monaten Lagerung bei 25°C und einer relativen Luftfeuchtigkeit von 60 % um jeweils maximal 5 Gew.-% verringert ist.

12. Verfahren nach einem der Ansprüche 1 bis 9, wobei der Gehalt an Proanthocyanidinen und 2H-1-Benzopyran-2-one nach 9 Monaten Lagerung bei 25°C und einer relativen Luftfeuchtigkeit von 60 % um jeweils maximal 3 Gew.-% verringert ist.

## Claims

1. Method for producing storage-stable solutions of pelargonium extracts, **characterised in that** the oxygen quantity at atmospheric pressure in the head space of the package used to store the solution of pelargonium extracts is reduced to a maximum of 0.025 parts by volume per volume part of the solution, wherein the content of proanthocyanidines and 2H-1-benzopyran-2-ones after 9 months of storage at 25°C and a relative humidity of 60 % is reduced by a maximum of 10 weight % each, said oxygen quantity being reduced
a) by gassing with an inert gas and/or b) by a reduced volume of the head space and/or c) by adding an oxygen removing agent,
wherein the oxygen removing agent contains one or more substances selected from the group consisting of ascorbic acid, salts of ascorbic acid, ascorbic acid derivatives, metals or metal salts in low oxidation states and oxidable polymers;
wherein the salts of the ascorbic acid are selected from the group comprising sodium ascorbate, potassium ascorbate and calcium ascorbate;
wherein the ascorbic acid derivatives are selected from the group comprising esters of ascorbic acid with fatty acids;
wherein the metals or metal salts in low oxidation states are selected from the group comprising iron, iron(II) oxide, iron(II) hydroxide and iron(II) chloride; and
wherein the oxidable polymer is a condensed polymer of m-xylylene diamine and adipic acid.

2. Method according to claim 1, wherein the 2H-1-benzopyran-2-ones are selected from 6,8-bis(sulfoxy)-7-hydroxy-2H-1-benzopyran-2-one (compound I) and 7-hydroxy-5,6-dimethoxy-8-sulfoxy-2H-1-benzopyran-2-one (compound II).

3. Method according to claim 1 or 2, wherein the inert gas is selected from the group consisting of nitrogen, noble gases such as argon, and carbon dioxide as well as mixtures of such gases.

4. Method according to claim 1, wherein the ascorbic acid derivatives are selected from the group comprising esters of ascorbic acid with palmitic or stearic acid.

5. Method according to any of the claims 1 to 4, wherein the pelargonium extract is recovered from *Pelargonium sidoides* and/or *Pelargonium reniforme*.

6. Method according to any of the claims 1 to 5, wherein the solution of pelargonium extracts is an aqueous solution or a solution in water and one or more alcohols and/or polyols.

7. Method according to claim 6, wherein the solution of pelargonium extracts is an aqueous-ethanolic solution, a solution in a mixture of water and glycerol or water, glycerol and ethanol.

8. Method according to any of the claims 1 to 7, wherein the oxygen quantity at atmospheric pressure in the head space of the package used for storing the solution of pelargonium extracts is reduced to a maximum of 0.015 parts by volume per volume part of the solution.

9. Method according to any of the claims 1 to 7, wherein the oxygen quantity at atmospheric pressure in the head space of the package used for storing the solution of pelargonium extracts is reduced to a maximum of 0.005 parts by volume per volume part of the solution.

10. Method according to any of the claims 1 to 9, wherein the content of proanthocyanidines and 2H-1-benzopyran-2-ones each is reduced by a maximum of 7 % by weight after 9 months of storage at 25°C and a relative humidity of 60 %.

11. Method according to any of the claims 1 to 9, wherein the content of proanthocyanidines and 2H-1-benzopyran-2-ones each is reduced by a maximum of 5 % by weight after 9 months of storage at 25°C and a relative humidity of 60 %.

12. Method according to any of the claims 1 to 9, wherein the content of proanthocyanidines and 2H-1-benzopyran-2-ones each is reduced by a maximum of 3 % by weight after 9 months of storage at 25°C and a relative humidity of 60 %.

## Revendications

1. Procédé de fabrication de solutions des extraits de Pélargonium stables au stockage **caractérisé en ce que** la teneur en oxygène à pression atmosphérique dans l'espace de tête du paquet utilisé pour le stockage de la solution des extraits de pélargonium est réduite au maximum à 0,025 parties de volume par 1 partie de volume de la solution, la teneur en proanthocyanidines et en 2H-1-benzopyran-2-ones étant réduite de 10% en poids au maximum, respectivement, après un stockage de 9 mois à 25°C sous une humidité relative de 60%,
- dans lequel la teneur en oxygène est réduite
a) par l'introduction d'un gaz protecteur et/ou
b) par un volume réduit de l'espace de tête et/ou
c) par l'addition d'un agent éliminant l'oxygène;
- dans lequel l'agent éliminant l'oxygène contient une ou plusieurs substances choisies dans le groupe consistant en l'acide ascorbique, les sels d' acide ascorbique, les dérivés d'acide ascorbique, les métaux ou les sels métalliques d'un degré d'oxydation inférieur et les polymères oxydables;
- dans lequel les sels d'acide ascorbique sont choisis dans le groupe comprenant l'ascorbate de sodium, l'ascorbate de potassium et l'ascorbate de calcium;
- dans lequel les dérivés d'acide ascorbique sont choisis dans le groupe comprenant les esters de l'acide ascorbique avec les acides gras;
- dans lequel les métaux et les sels métalliques d'un degré d'oxydation inférieur sont choisis dans le groupe comprenant le fer, l'oxyde ferreux, l'hydroxyde ferreux et le chlorure ferreux et
- dans lequel le polymère oxydable est un polymère condensé constitué de meta-xylylène diamine et d'acide adipique.

2. Procédé selon la revendication 1, dans lequel les 2H-1-benzopyran-2-ones sont choisis parmi le 6,8-bis(sulfoxy)-7-hydroxy-2H-1-benzopyran-2-one (composé 1) et le 7-hydroxy-5,6-diméthoxy-8-sulfoxy-2H-1-benzopyran-2-one (composé II).

3. Procédé selon la revendication 1 ou 2, dans lequel le gaz protecteur est choisi dans le groupe consistant en l'azote, les gaz nobles, p.e. l'argon, et le dioxyde de carbone ainsi que les mélanges de ces gazes.

4. Procédé selon la revendication 1, dans lequel les dérivés d'acide ascorbique sont choisis dans le groupe comprenant les esters de l'acide ascorbique avec l'acide palmitique ou l'acide stéarique.

5. Procédé selon l'une quelconque des revendications 1 à 4, dans lequel l'extrait de pélargonium est obtenu à partir de pélargonium sidoides et/ou pélargonium reniforme.

6. Procédé selon l'une quelconque des revendications 1 à 5, dans lequel la solution des extraits de pélargonium est une solution aqueuse ou une solution dans l'eau et un ou plusieurs alcools et/ou polyoles.

7. Procédé selon la revendication 6, dans lequel la solution des extraits de pélargonium est une solution aqueuse éthanolique, une solution dans un mélange de l'eau et de la glycérine ou de l'eau, de la glycérine et de l'éthanol.

8. Procédé selon l'une quelconque des revendications 1 à 7, dans lequel la teneur en oxygène à pression atmosphérique dans l'espace de tête de paquet utilisé pour le stockage de la solution des extraits de pélargonium est réduite au maximum à 0,015 parties de volume par 1 partie de volume de la solution.

9. Procédé selon l'une quelconque des revendications 1 à 7, dans lequel la teneur en oxygène à pression atmosphérique dans l'espace de tête de paquet utilisé pour le stockage de la solution des extraits de pélargonium est réduite au maximum à 0,005 parties de volume par 1 partie de volume de la solution.

10. Procédé selon l'une quelconque des revendications 1 à 9, dans lequel la teneur en proanthocyanidines et en 2H-1-benzopyran-2-one est réduite de 7% en poids au maximum, respectivement, après un stockage de 9 mois à 25°C sous une humidité relative de 60%.

11. Procédé selon l'une quelconque des revendications 1 à 9, dans lequel la teneur en proanthocyanidines et en 2H-1-benzopyran-2-one est réduite de 5% en poids au maximum, respectivement, après un stockage de 9 mois à 25°C sous une humidité relative de 60%.

12. Procédé selon l'une quelconque des revendications 1 à 9, dans lequel la teneur en proanthocyanidines et en 2H-1-benzopyran-2-one est réduite de 3% en poids au maximum, respectivement, après un stockage de 9 mois à 25°C sous une humidité relative de 60%.
